# EUROPEAN PATENT SPECIFICATION

(11) **EP 4 329 866 B1**
(45) Date of publication and mention of the grant of the patent: **05.11.2025**
(21) Application number: 21730757.8
(22) Date of filing: 14.05.2021
(51) Int. Cl.: A61M 39/02, A61M 39/12, A61M 25/01, A61M 39/10

(54) **PORT WITH DETACHABLE STEM**
PORT MIT ABNEHMBAREM SCHAFT
ORIFICE À TIGE DÉTACHABLE

(43) Date of publication of application: 06.03.2024
(73) Proprietor: Bard Peripheral Vascular, Inc., Franklin Lakes, NJ 07417 (US)
(72) Inventor: DENSLEY, Bryon, Ray, Fremont, CA 94538 (US); FIUMEFREDDO, Diana, Salt Lake City, UT 84121 (US); THOMAS, Ian, N., West Bountiful, UT 84087 (US); HOYE, Jessica, Phoenix, AZ 85032 (US); ANDERSEN, Christian, Kaysville, UT 84037 (US)
(74) Representative: Hoffmann Eitle
(86) International application number: PCT/US2021/032615
(87) International publication number: WO 2022/240421

(56) References cited:
- AU-A1- 2019 338 124
- US-A1- 2007 149 947
- US-B2- 9 889 255

## Description

### BACKGROUND

US 9,889,255 B2 discloses a needle assembly including a needle and a needle shield device. The needle shield device includes an inner shield connectable to at least one a base and a fluid connector connectable to the base, and an outer shield fixedly connected to the opposing end the needle and displaceable relative to the inner shield between a first position, in which the sharpened end of the needle is exposed outside the inner shield, and a second position, in which the sharpened end of the needle is shielded by the inner shield. Interaction between portions of the inner and outer shields during or after proximal displacement of the outer shield from the first position to the second position automatically releases or permits release of the inner shield from connection to the at least one of the base and the fluid connector.

US 2007/0149947 A1 discloses a rotational locking mechanism for securing a catheter to a surgical implant.

### SUMMARY

Briefly summarized, embodiments disclosed herein are directed to a port system with a detachable stem and associated methods thereof.

Proximally trimmable catheters allow for post-placement sizing of the catheter. When placing a catheter and port assembly, the position of the distal tip of the catheter can be important for the efficacy of the treatment. For example, when placing a catheter within the superior vena cava, if the distal tip of the catheter falls short of the target area, the efficacy of the medicament is reduced. If the distal tip is advanced too far, the distal tip can cause arrhythmia. The distance between the distal tip of the catheter and the port can vary since the distances between the target location, insertion site to the vasculature, and the location of the port can vary between patients and procedures. Estimating the catheter length before placement can lead to errors that result in misplacement of the distal tip.

Proximally trimmable catheters allow for placement of the catheter distal tip at the target location before trimming a proximal portion of the catheter to the correct length. The clinician can then attach the catheter to a subcutaneous port, or similar access device. However, securing the catheter to the port can be challenging. The connection must be leak-proof, especially under high-pressure infusion. Further, manipulating the catheter and port within the confined, wetted environment of a subcutaneous access site can lead to slippage, undue trauma to the access site, or misplacement of the catheter distal tip.

Embodiments disclosed herein are directed to a port system including a connector that can be coupled to the catheter prior to coupling the connector to the port. The connector can be secured to the trimmed end of the catheter. The connector can then facilitate engagement with the port in a fluid tight connection.

Disclosed herein is a vascular access system including, a port defining a reservoir configured to be accessed percutaneously by a needle and including a port lumen in fluid communication with the reservoir, and a connector having a stem defining a stem lumen extending along a longitudinal axis, the connector slidably engaged with the port in a first direction along an axis extending perpendicular to the longitudinal axis and transitionable between a disengaged position and an engaged position. The vascular access system further includes a locking pin, slidably engaged with the connector along the longitudinal axis between an unlocked position and a locked position

In some embodiments, the stem lumen aligns with the port lumen to provide fluid communication therebetween when the connector is in the engaged position. In some embodiments, the port includes a recess configured to receive a body of the connector therein, when in the engaged position. In some embodiments, the connector body engages the recess in one of an interference fit, press-fit, or snap-fit engagement. In some embodiments, the port includes a channel and the connector includes a rail, the rail configured to slidably engage the channel as the connector engages the port. In some embodiments, the rail engages the channel in one of an interference fit, press-fit, or snap-fit engagement.

In some embodiments, wherein the connector includes an abutment surface configured to engage a surface of the port to inhibit further movement in the first direction, when the connector is in the engaged position. In some embodiments, the axis extending perpendicular to the longitudinal axis is one of a transverse axis, lateral axis, or an axis extending at an angle therebetween. In some embodiments, the locking pin engages a locking aperture, disposed in the port, when in the locked position.

In some embodiments, the locking pin engages one of the connector or the port in one of an interference fit, press-fit, or snap-fit engagement. In some embodiments, the locking pin in the locked position provides a smooth outer profile to the connector. In some embodiments, the port includes a needle penetrable septum disposed over the reservoir and configured to provide percutaneous access to the reservoir. In some embodiments, a surface of the connector that the stem extends from, forms a continuous outer profile with a surface of the port when the connector is in the engaged position.

Also disclosed is a method of coupling a catheter to a port including, urging a stem into a lumen of the catheter, the stem extending from a connector body and defining a stem lumen extending along a longitudinal axis, aligning the connector body with a recess disposed on a port, along an axis extending perpendicular to the longitudinal axis, slidably engaging the connector body with the recess in a first direction along the axis extending perpendicular to the longitudinal axis, aligning the stem lumen with a port lumen to provide fluid communication between the catheter and the port.

In some embodiments, an abutment surface of the connector body contacts a surface of the recess when the stem lumen is aligned with the port lumen, to inhibit further movement in the first direction. In some embodiments, the connector body engages the recess in one of a press-fit, interference fit, or snap-fit engagement. In some embodiments, the method further includes slidably engaging a rail disposed on the connector body with a channel deposed in the recess. In some embodiments, the rail engages the channel in one of a press-fit, interference fit, or snap-fit engagement. In some embodiments, the method further includes sliding a locking pin from an unlocked position to a locked position, when the stem lumen is aligned with the port lumen, to inhibit movement of the connector body in one of the first direction, or a second direction opposite the first direction.

In some embodiments, the locking pin is slidably engaged with the connector body. In some embodiments, the locking pin engages a locking aperture disposed in the recess, when in the locked position. In some embodiments, the locking pin engages one of the connector body or the locking aperture in one of a press-fit, interference fit, or snap-fit engagement. In some embodiments, the locking pin in the locked position forms a smooth outer profile with the connector body. In some embodiments, the port includes a reservoir in fluid communication with the port lumen, and a needle penetrable septum configured to provide access to the reservoir.

### DRAWINGS

A more particular description of the present disclosure will be rendered by reference to specific embodiments thereof that are illustrated in the appended drawings. It is appreciated that these drawings depict only typical embodiments of the invention and are therefore not to be considered limiting of its scope. Example embodiments of the invention will be described and explained with additional specificity and detail through the use of the accompanying drawings in which:
FIG. 1 shows an exploded, perspective view of a port connector system, in accordance with embodiments disclosed herein.
FIG. 2A shows a perspective view of a port, in accordance with embodiments disclosed herein.
FIG. 2B shows a perspective view of a connector, in accordance with embodiments disclosed herein.
FIG. 3 shows a perspective view of a port connector system, in accordance with embodiments disclosed herein.
FIGS. 4A-4F show an exemplary method of use for a port connector system, in accordance with embodiments disclosed herein.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal end portion" of, for example, a catheter disclosed herein includes a portion of the catheter intended to be near a clinician when the catheter is used on a patient. Likewise, a "proximal length" of, for example, the catheter includes a length of the catheter intended to be near the clinician when the catheter is used on the patient. A "proximal end" of, for example, the catheter includes an end of the catheter intended to be near the clinician when the catheter is used on the patient. The proximal portion, the proximal end portion, or the proximal length of the catheter can include the proximal end of the catheter; however, the proximal portion, the proximal end portion, or the proximal length of the catheter need not include the proximal end of the catheter. That is, unless context suggests otherwise, the proximal portion, the proximal end portion, or the proximal length of the catheter is not a terminal portion or terminal length of the catheter.

With respect to "distal," a "distal portion" or a "distal end portion" of, for example, a catheter disclosed herein includes a portion of the catheter intended to be near or in a patient when the catheter is used on the patient. Likewise, a "distal length" of, for example, the catheter includes a length of the catheter intended to be near or in the patient when the catheter is used on the patient. A "distal end" of, for example, the catheter includes an end of the catheter intended to be near or in the patient when the catheter is used on the patient. The distal portion, the distal end portion, or the distal length of the catheter can include the distal end of the catheter; however, the distal portion, the distal end portion, or the distal length of the catheter need not include the distal end of the catheter. That is, unless context suggests otherwise, the distal portion, the distal end portion, or the distal length of the catheter is not a terminal portion or terminal length of the catheter.

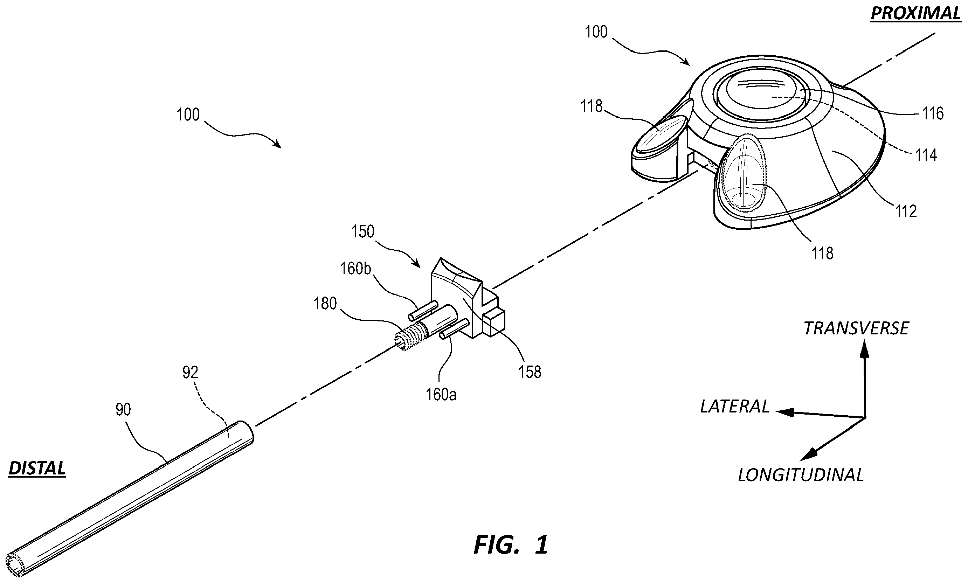

To assist in the description of embodiments described herein, as shown in FIG. 1, a longitudinal axis extends substantially parallel to an axial length of the catheter. A lateral axis extends normal to the longitudinal axis, and a transverse axis extends normal to both the longitudinal and lateral axes.

Unless defined otherwise, all technical and scientific terms used herein have the same meaning as commonly understood by those of ordinary skill in the art.

FIG. 1 shows an exploded, perspective view of a port system ("system") 100 generally including a port 110, and a detachable connector 150 including a port stem 180, configured to be coupled to a catheter 90. In an embodiment, the port 110 can include a body 112 defining a reservoir 114 and include a needle penetrable septum 116, disposed thereover. In use, the port 110 can be disposed subcutaneously. The reservoir 114 of the port 110 can be accessed by a needle extending percutaneously through the needle-penetrable septum 116. A port lumen 120 can provide fluid communication between the reservoir 114 and the port stem 180. As will be appreciated, embodiments disclosed herein can be used with single reservoir ports or multi-reservoir ports, without limitation. In an embodiment, the port 110 can include a suture aperture 118 configured to receive a suture therethrough securing the port 110 within a tissue pocket. Optionally the suture aperture 118 can include a needle penetrable material, e.g. silicone, or the like, disposed therein to mitigate tissue ingrowth.

In an embodiment, a connector 150 can include a connector body 152 having the port stem 180 extending therefrom along a longitudinal axis, and defining a stem lumen 154. The stem lumen 154 can extend through the port stem 180 and through the connector body 152 and can align with the port lumen 120 when the connector 150 is fully engaged with the port 110.

In an embodiment, the catheter 90 can include a compliant elongate tube configured to engage the port stem 180 in an interference fit. A distal end of the catheter 90 can be disposed within a vasculature of a patient to provide fluid communication therewith. A proximal end of the catheter 90 can be trimmable and configured to be stretched over the port stem 180 to provide a fluid-tight seal there between. Worded differently, the stem 180 can be configured to engage an inner lumen of the catheter 90. The catheter 90 can be elastically deformed to receive the port stem 120 therein. In an embodiment, a cathlock (not shown) can engage an outer surface of the catheter 90 and can further secure the catheter 90 to the port stem 180. In an embodiment, the port stem 180 can include barbs, ridges, or similar structures extending annularly about the stem 180 and configured to retain the catheter 90 disposed thereon.

In an embodiment the connector body 152 can slidably engage the port body 112 along an axis extending perpendicular to the longitudinal axis, securing the connector body 152 thereto and aligning the stem lumen 154 with the port lumen 120. In an embodiment, the connector body 152 can slidably engage the port body 112 along a transverse axis, a lateral axis, or an axis extending at an angle therebetween. In an embodiment, one of the port lumen 120 or the stem lumen 154 can include a gasket disposed at the interface between the port lumen 120 or the stem lumen 154 to provide a fluid tight seal therebetween, when the port lumen 120 is aligned with the stem lumen 154.

In an embodiment, the port body 110 can include a recess 122 configured to receive a portion of the connector body 152 therein. In an embodiment, the connector body 152 can engage the recess 122 in one of a press-fit or interference fit engagement and configured to mitigate retrograde movement of the connector body 152 from the recess 122, preventing accidental disengagement. In an embodiment, one of the connector body 152 or the recess 122 can include a protrusion, detent, pawl, clip, barb, latch or similar mechanism to allow the connector body 152 to engage the recess 122 in a snap-fit engagement. Advantageously, the snap-fit engagement can provide an audible confirmation that the connector body 152 is fully engaged with the recess 122. Further, the snap-fit engagement can mitigate retrograde movement of the connector body 152 from the recess 122, preventing accidental disengagement.

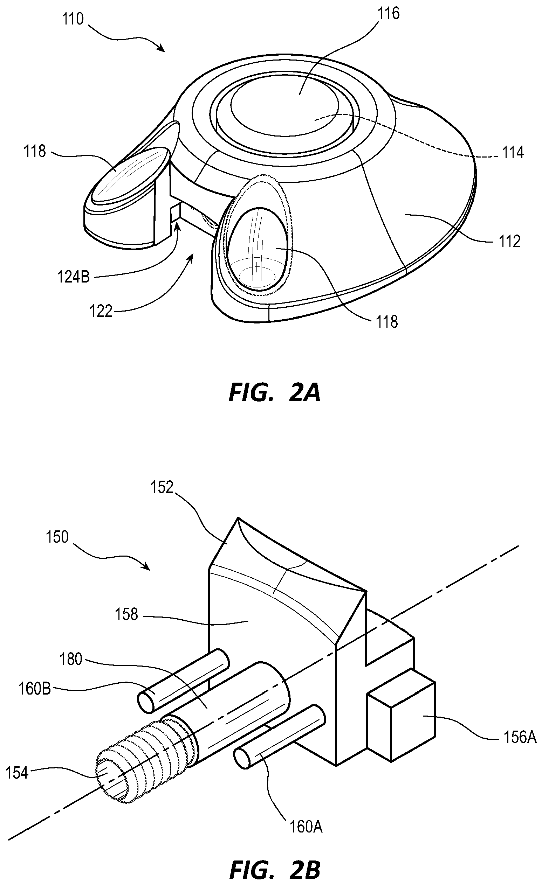

In an embodiment, as shown in FIG. 2A, the recess 122 can include one or more channels 124 disposed in a side wall of the recess 122 and aligned with the axis of movement of the connector body 152 relative to the port body 112, e.g. a transverse axis. In an embodiment, as shown in FIG. 2B, the connector body 152 can include a rail 156 extending laterally therefrom and extending along the same axis as the channel, e.g. a transverse axis. The rail 156 can be configured to slidably engage the channel 124, along the axis, e.g. the transverse axis, as the connector body 152 slidably engages the recess 122. As will be appreciated, in an embodiment where the connector body 152 slidably engages the recess 122 along a lateral axis, the rail 156 and the channel 124 can also extend along the lateral axis.

In an embodiment, the connector body 152 can include a first rail 156A extending from a first lateral side and a second rail 156B extending from a second lateral side, opposite the first side. In an embodiment, the recess 122 can include a first channel 124A disposed in a first wall of the recess 122, and a second channel 124B disposed in a second wall of the recess 122, opposite the first channel 124A. The first rail 156A can slidably engage the first channel 124A and the second rail 156B can slidably engage the second channel 124B along an axis, e.g. the transverse axis.

In an embodiment, the rail 156 can engage the channel 124 in one of a press-fit or interference fit engagement to mitigate retrograde movement of the connector body 152 from the recess 122. In an embodiment, one of the rail 156 or the channel 124 can include a protrusion, detent, pawl, barb, clip, latch or similar mechanism to engage the rail 156 with the channel 124 in a snap-fit engagement, as described herein. In an embodiment, the rail 156 can be disposed on a wall of the recess 122 and can be configured to slidably engage a channel 124 disposed on the connector body 152, as described herein. Advantageously, the rail 156 can engage the channel 124 to inhibit longitudinal movement of the connector 150 relative to the port 110 when the connector body 152 is engaged with the recess 122.

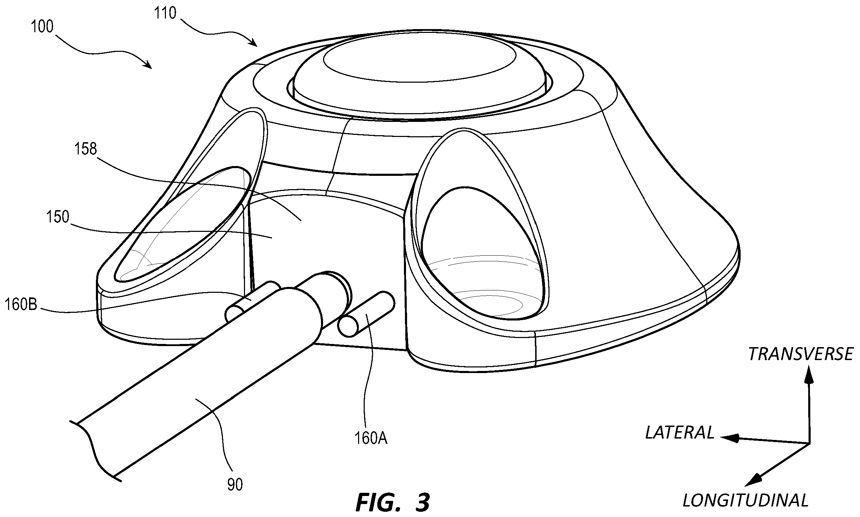

In an embodiment, with the connector 150 engaged with the port 110, one or more surfaces of the connector body 152, e.g. a stem surface 158, can co-operate with the port body 112 to define a continuous outer profile (FIG. 3). Advantageously, the continuous outer profile of the port 110 and connector 150 assembly can mitigate tissue ingrowth and improve patient comfort. In an embodiment, a top surface of the connector body 152 can abut against a surface of the recess 122 to inhibit further movement through the recess 122. In an embodiment, the top surface can abut against the surface of the recess 122 when the connector body 152 is fully engaged with the recess 122, i.e. when the port lumen 120 is aligned with the stem lumen 154.

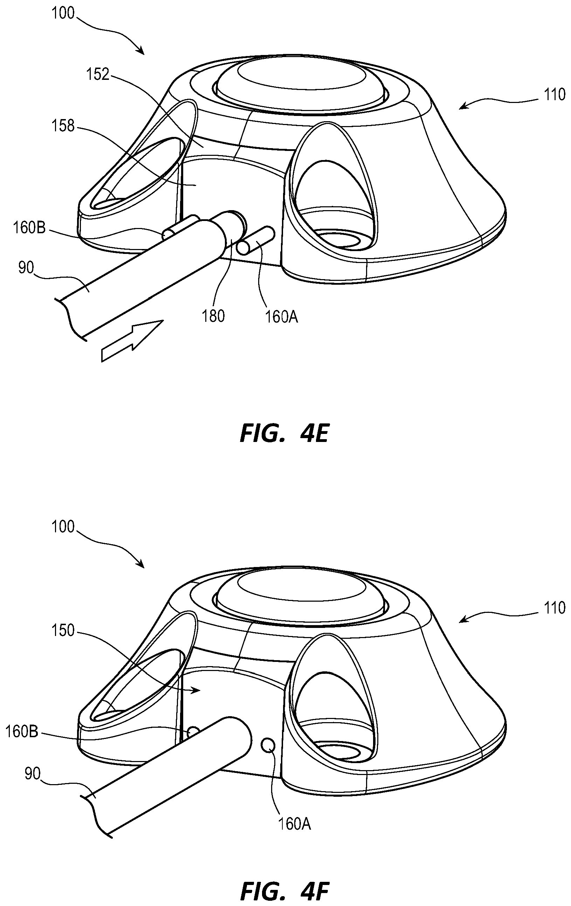

In an embodiment, the connector 150 can include a locking pin 160, for example a first locking pin 160A and a second locking pin 160B. The locking pin 160 can be slidably engaged with the connector body 150 along a longitudinal axis, i.e. perpendicular to the axis along which the connector body 152 is slidably engaged with the recess 122. As shown, the locking pins 160 are slidably engaged along the longitudinal axis. However, it will be appreciated that other axes are also contemplated. The locking pins 160 can transition between an unlocked position (FIG. 2B) and a locked position (FIG. 4F).

In an embodiment, when the connector body 152 is fully engaged with the recess 122, i.e. when the stem lumen 154 aligns with the port lumen 120, each of the locking pins 160 can align with a locking aperture 162. For example, a first locking pin 160A can align with a first locking aperture 162A, and a second locking pin 160B can align with a second locking aperture 162B. As will be appreciated, various numbers, locations and configurations of locking pin(s) 160 and locking aperture(s) 162 are contemplated to fall within the scope of the present invention.

In an embodiment, the locking pin 160 can engage one of the connector body 152 or the locking aperture 162 in a press-fit or interference fit engagement configured to mitigate retrograde movement of the locking pin 160 from the locking aperture 162 and preventing accidental disengagement. In an embodiment, the locking pin 160 and locking aperture 162 can include a protrusion, detent, pawl, barb, clip, latch, or the like, configured to provide a snap-fit engagement therebetween. Advantageously, the snap-fit engagement can provide an audible indication of when the locking pin is in the locked position. Further the snap-fit engagement can mitigate retrograde movement of the locking pin 160 from the locking aperture 162 preventing accidental disengagement.

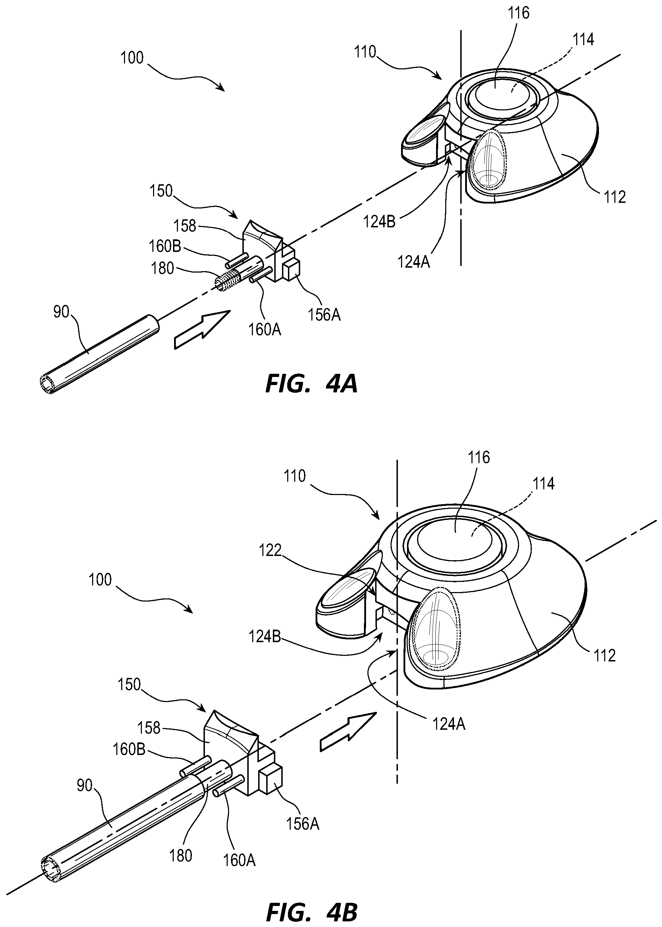

FIGS. 4A-4F show an exemplary method of use for a port system 100 including a port 110 and connector 150 having a stem 180 extending therefrom, as described herein. Optionally a distal tip of a catheter 90 can be disposed at a target location within a vasculature of the patient. A proximal end of the catheter 90 can then be trimmed to a suitable length. Optionally the port 110 can be disposed within a tissue pocket.

As shown in FIGS. 4A-4B, in an embodiment, the clinician can then couple the stem 180 of the connector 150 with the proximal end of the catheter 90 by urging the stem into a lumen 92 of the catheter 90. The catheter 90 can elastically deform radially outward to receive the stem therein and can be secured thereto in an interference fit engagement. Optionally, a cathlock or similar structure (not shown) can engage an outer surface of the catheter 90 to further secure the catheter 90 to the stem 180.

Advantageously, the connector 150 can be coupled to the catheter 90 outside of the tissue pocket, e.g. externally to the patient. This can provide the clinician with greater range of motion, or leverage, facilitating securing the catheter 90 to the stem 180. Further, the clinician can grasp the catheter 90 and connector 150 outside of the wetted environment of the tissue pocket providing increased grip. Advantageously, coupling the connector 150 to the catheter 90 externally to the patient can provide increase space to use tools for grasping one of the catheter 90 or the connector 150 facilitating the engagement.

As shown in FIGS. 4B-4D, with the stem 180 engaged with the catheter 90, the connector body 152 can be coupled to the port 110. Optionally, the connector 150 can be placed within the tissue pocket and coupled to the port 110 within the tissue pocket. The connector body 152 can be aligned with the recess 122 disposed on the port 110 along an axis extending perpendicular to the longitudinal axis. In an embodiment, the connector body 152 can be aligned with the recess 122 along a transverse axis. In an embodiment, a rail 156 of the connector body 152 can be aligned with a channel 124 of the recess 122 along a transverse axis. For example, a first rail 156A can be aligned with a first channel 124A, and a second rail 156B can be aligned with a second channel 124B. Advantageously, the transverse axis engagement can minimize lateral movement within the tissue pocket, providing a "tighter" tissue pocket, and mitigating subcutaneous travel once the port has been placed.

In an embodiment, the connector body 152 can be aligned with the recess 122 along a lateral axis. Advantageously, the lateral axial engagement allows the connector 150 and the port 110 to both be aligned within the tissue pocket before engagement. Further, the clinician can apply a lateral "pinching" action to couple the connector 150 to the port 110, without applying direct pressure to the patient.

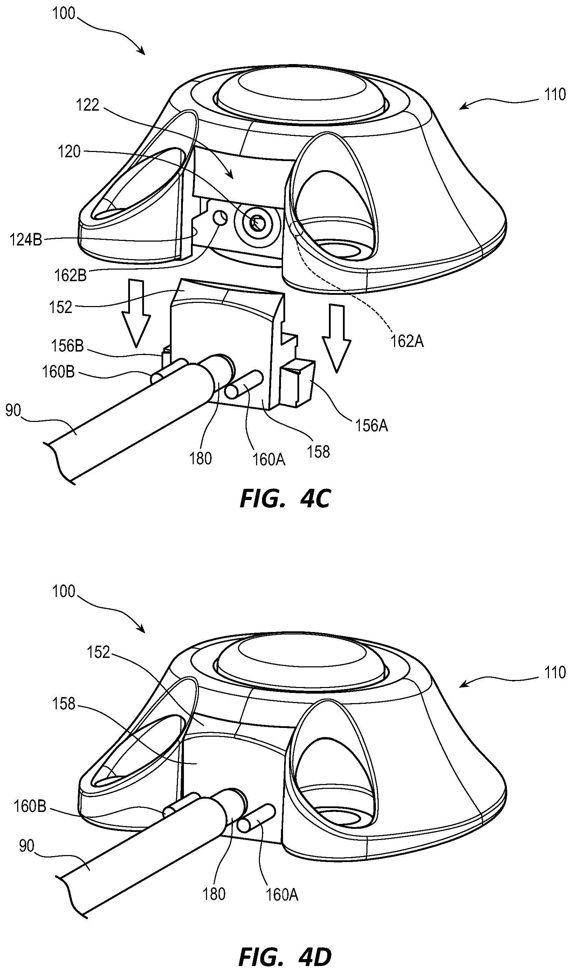

As shown in FIGS. 4C-4D, the connector body 152 can be disposed below the recess 122 along a transverse axis. In an embodiment, the connector body 152 can be slid transversely upwards, in a first direction, into the recess 122. In an embodiment, the port 110 can be slid transversely downwards, in a second direction, onto the connector body 152. As the connector body 152 slidably engages the recess 122, the rail 156 can engage the channel 124. The connector body 152 can continue to slidably engage the recess 122 until a top surface of the connector body 152 abuts against a surface, e.g. a downward facing surface, of the recess 122. Advantageously, the connector 150 engaging the port in an upwards direction can allow the port 110 to be sutured in place against the floor of the tissue pocket and prevent accidental disengagement of the connector 150 from the port 110 in a second direction, i.e. downwards.

As will be appreciated, in an embodiment, the connector body 152 can slidably engage the recess 122 along a transverse axis in a downwards direction, i.e. a second direction. As such, a bottom surface of the connector body 152 can abut against an upward facing surface of the recess 122 preventing further transverse movement of the connector body 152. In an embodiment, an end surface of the rail 156 can abut against an end surface of the channel 124 to prevent further transverse movement, when the connector body 152 is fully engaged with the port 110.

In an embodiment, the connector body 152 can be slidably engaged with the recess 122 along a lateral axis. The connector body 152 can engage the recess 122 in either a first lateral direction, or a second lateral direction opposite the first direction and can include one or more abutments, as described herein, configured to inhibit further lateral movement when the connector body 152 is fully engaged with the recess 122, i.e. when a stem lumen 154 is aligned with a port lumen 120.

As shown in FIG. 4D, with the connector body 152 fully engaged with the recess 122, the stem lumen 154 is aligned with a port lumen 120 providing fluid communication therebetween and providing fluid communication between the port reservoir 114 and the catheter lumen 92. In an embodiment, one or more surfaces of the connector body 152, e.g. a stem surface 158 can co-operate with an outer surface of the port 110 to define a continuous outer surface. Advantageously, the continuous outer surface can mitigate tissue ingrowth and provide a smooth outer profile, improving patient comfort.

As shown in FIGS. 4E-4F, in an embodiment, the locking pin(s) 160 can be transitioned from an unlocked position (FIG. 4E) to a locked position (FIG. 4F). The locking pin(s) 160 can slide along a longitudinal axis, i.e. along an axis extending perpendicular to the axis along which the connector body 152 engages the recess 122. The locking pin(s) 160 in the locked position can engage the locking aperture(s) 162 and can inhibit retrograde movement of the connector body 152 from the recess 122. In an embodiment, the locking pin 160 can engage one of the connector body 152 or the locking aperture 162 in an interference fit to secure the locking pin(s) 160 in one of the unlocked or locked position.

In an embodiment, the port system 100 can include various configurations of connectors 150 having different stem sizes or morphology. Each connector 150 can then be coupled with the port 110 as described herein. For example, various types of connectors 150 can include different stem size or morphology configured to couple with different types of catheter, i.e. catheters that differ in size, number or shape of lumen, material, flow capacities, or the like etc. or stems configured to engage different cathlock structures. The connector 150 can then be coupled to the port 110 as described herein. As such, a single port configuration, e.g. port 110, can be coupled to various different types of catheter, where only the connector 150 would need to be exchanged. Advantageously, such a system can provide improved efficiency in inventory offerings, storage, and manufacturing, as well as reducing associated costs.

While some particular embodiments have been disclosed herein, and while the particular embodiments have been disclosed in some detail, it is not the intention for the particular embodiments to limit the scope of the concepts provided herein. Additional adaptations and/or modifications can appear to those of ordinary skill in the art, and, in broader aspects, these adaptations and/or modifications are encompassed as well. Accordingly, departures may be made from the particular embodiments disclosed herein without departing from the scope of the concepts provided herein.

## Claims

1. A vascular access system (100), comprising:
a port (110) defining a reservoir (114) configured to be accessed percutaneously by a needle and including a port lumen (120) in fluid communication with the reservoir (114); and
a connector (150) having a stem (180) defining a stem lumen (154) extending along a longitudinal axis, the connector (150) slidably engaged with the port (110) in a first direction along an axis extending perpendicular to the longitudinal axis and transitionable between a disengaged position and an engaged position,
**characterised in that** the vascular access system comprises a locking pin (160), slidably engaged with the connector (150) along the longitudinal axis between an unlocked position and a locked position.

2. The vascular access system according to claim 1, wherein the stem lumen (154) aligns with the port lumen (120) to provide fluid communication therebetween when the connector (150) is in the engaged position.

3. The vascular access system according to any one of claims 1-2, wherein the port (110) includes a recess (122) configured to receive a body (152) of the connector (150) therein, when in the engaged position.

4. The vascular access system according to claim 3, wherein the connector body (152) engages the recess (122) in one of an interference fit, press-fit, or snap-fit engagement.

5. The vascular access system according to any one of claims 1-4, wherein the port (110) includes a channel (124) and the connector (150) includes a rail (156), the rail configured to slidably engage the channel as the connector engages the port.

6. The vascular access system according to claim 5, wherein the rail (156) engages the channel (124) in one of an interference fit, press-fit, or snap-fit engagement.

7. The vascular access system according to any one of claims 1-6, wherein the connector (150) includes an abutment surface configured to engage a surface of the port (110) to inhibit further movement in the first direction, when the connector (150) is in the engaged position.

8. The vascular access system according to any one of claims 1-7, wherein the axis extending perpendicular to the longitudinal axis is one of a transverse axis, lateral axis, or an axis extending at an angle therebetween.

9. The vascular access system according to any one of claims 1-8, wherein the locking pin (160) engages a locking aperture (162), disposed in the port, when in the locked position.

10. The vascular access system according to any one of claims 1-9, wherein the locking pin (160) engages one of the connector (150) or the port (110) in one of an interference fit, press-fit, or snap-fit engagement.

11. The vascular access system according to any one of claims 1-10, wherein the locking pin (160) in the locked position provides a smooth outer profile to the connector (150).

12. The vascular access system according to any one of claims 1-11, wherein the port (110) includes a needle penetrable septum (116) disposed over the reservoir (114) and configured to provide percutaneous access to the reservoir (114).

13. The vascular access system according to any one of claims 1-12, wherein a surface of the connector (150) that the stem extends from, forms a continuous outer profile with a surface of the port (110) when the connector is in the engaged position.

## Patentansprüche

1. Vaskuläres Zugangssystem (100), umfassend:
einen Port (110), der ein Reservoir (114) umgrenzt, das so konfiguriert ist, dass es perkutan durch eine Nadel zugänglich ist, und ein Portlumen (120) in Fluidverbindung mit dem Reservoir (114) einschließt; und
ein Verbindungsstück (150) mit einem Schaft (180), der ein Schaftlumen (154) umgrenzt, das sich entlang einer Längsachse erstreckt, wobei das Verbindungsstück (150) in einer ersten Richtung entlang einer Achse, die sich senkrecht zu der Längsachse erstreckt, verschiebbar mit dem Port (110) in Eingriff steht und zwischen einer nicht in Eingriff stehenden Position und einer in Eingriff stehenden Position übergehen kann,
**dadurch gekennzeichnet, dass** das vaskuläre Zugangssystem einen Verriegelungsstift (160) umfasst, der mit dem Verbindungsstück (150) entlang der Längsachse zwischen einer entriegelten Position und einer verriegelten Position verschiebbar in Eingriff steht.

2. Vaskuläres Zugangssystem nach Anspruch 1, wobei das Schaftlumen (154) mit dem Portlumen (120) fluchtet, um eine Fluidverbindung dazwischen herzustellen, wenn sich das Verbindungsstück (150) in der in Eingriff stehenden Position befindet.

3. Vaskuläres Zugangssystem nach einem der Ansprüche 1-2, wobei der Port (110) eine Ausnehmung (122) einschließt, die so konfiguriert ist, dass sie einen Körper (152) des Verbindungsstücks (150) darin aufnimmt, wenn dieses sich in der in Eingriff stehenden Position befindet.

4. Vaskuläres Zugangssystem nach Anspruch 3, wobei der Verbindungsstück-Körper (152) in die Ausnehmung (122) in einem von einer Presspassung, Presssitz oder Schnapppassung eingreift.

5. Vaskuläres Zugangssystem nach einem der Ansprüche 1-4, wobei der Port (110) einen Kanal (124) einschließt und das Verbindungsstück (150) eine Schiene (156) einschließt, wobei die Schiene so konfiguriert ist, dass sie gleitend mit dem Kanal in Eingriff steht, wenn das Verbindungsstück mit dem Port in Eingriff steht.

6. Vaskuläres Zugangssystem nach Anspruch 5, wobei die Schiene (156) mit dem Kanal (124) in einem von einer Presspassung, Presssitz oder Schnapppassung in Eingriff steht.

7. Vaskuläres Zugangssystem nach einem der Ansprüche 1-6, wobei das Verbindungsstück (150) eine Anschlagfläche einschließt, die so konfiguriert ist, dass sie mit einer Fläche des Ports (110) in Eingriff kommt, um eine weitere Bewegung in der ersten Richtung zu verhindern, wenn sich das Verbindungsstück (150) in der in Eingriff stehenden Position befindet.

8. Vaskuläres Zugangssystem nach einem der Ansprüche 1-7, wobei die Achse, die sich senkrecht zu der Längsachse erstreckt, eine von einer Querachse, einer Seitenachse oder eine Achse ist, die sich in einem Winkel dazwischen erstreckt.

9. Vaskuläres Zugangssystem nach einem der Ansprüche 1-8, wobei der Verriegelungsstift (160) in der verriegelten Position in eine in dem Port angeordnete Verriegelungsöffnung (162) eingreift.

10. Vaskuläres Zugangssystem nach einem der Ansprüche 1-9, wobei der Verriegelungsstift (160) entweder mit dem Verbindungsstück (150) oder dem Port (110) in einer Presspassung, einem Presssitz oder einer Schnapppassung in Eingriff steht.

11. Vaskuläres Zugangssystem nach einem der Ansprüche 1-10, wobei der Verriegelungsstift (160) in der verriegelten Position dem Verbindungsstück (150) ein glattes Außenprofil verleiht.

12. Vaskuläres Zugangssystem nach einem der Ansprüche 1-11, wobei der Port (110) ein mit einer Nadel durchdringbares Septum (116) einschließt, das über dem Reservoir (114) angeordnet und so konfiguriert ist, dass es einen perkutanen Zugang zu dem Reservoir (114) ermöglicht.

13. Vaskuläres Zugangssystem nach einem der Ansprüche 1-12, wobei eine Fläche des Verbindungsstücks (150), von der sich der Schaft erstreckt, ein durchgehendes Außenprofil mit einer Fläche des Ports (110) bildet, wenn sich das Verbindungsstück in der in Eingriff stehenden Position befindet.

## Revendications

1. Système d'accès vasculaire (100), comprenant :
un orifice (110) définissant un réservoir (114) configuré pour être accessible par voie percutanée par une aiguille et incluant une lumière d'orifice (120) en communication fluidique avec le réservoir (114) ; et
un connecteur (150) présentant une tige (180) définissant une lumière de tige (154) s'étendant le long d'un axe longitudinal, le connecteur (150) étant engagé de manière coulissante avec l'orifice (110) dans une première direction le long d'un axe s'étendant perpendiculairement à l'axe longitudinal et pouvant passer d'une position désengagée à une position engagée, **caractérisé en ce que** le système d'accès vasculaire comprend
une goupille de verrouillage (160), engagée de manière coulissante avec le connecteur (150) le long de l'axe longitudinal entre une position déverrouillée et une position verrouillée.

2. Système d'accès vasculaire selon la revendication 1, dans lequel la lumière de tige (154) s'aligne avec la lumière d'orifice (120) pour fournir une communication fluidique entre celles-ci lorsque le connecteur (150) est dans la position engagée.

3. Système d'accès vasculaire selon l'une quelconque des revendications 1 à 2, dans lequel l'orifice (110) inclut un évidement (122) configuré pour recevoir un corps (152) du connecteur (150) à l'intérieur, lorsqu'il est dans la position engagée.

4. Système d'accès vasculaire selon la revendication 3, dans lequel le corps (152) de connecteur s'engage dans l'évidement (122) selon un engagement parmi un ajustement avec serrage, un ajustement forcé ou un encliquetage.

5. Système d'accès vasculaire selon l'une quelconque des revendications 1 à 4, dans lequel l'orifice (110) inclut un canal (124) et le connecteur (150) inclut un rail (156), le rail étant configuré pour s'engager de manière coulissante dans le canal lorsque le connecteur s'engage dans l'orifice.

6. Système d'accès vasculaire selon la revendication 5, dans lequel le rail (156) s'engage dans le canal (124) selon un engagement parmi un ajustement avec serrage, un ajustement forcé ou un encliquetage.

7. Système d'accès vasculaire selon l'une quelconque des revendications 1 à 6, dans lequel le connecteur (150) inclut une surface de butée configurée pour s'engager dans une surface de l'orifice (110) pour empêcher tout mouvement supplémentaire dans la première direction, lorsque le connecteur (150) est dans la position engagée.

8. Système d'accès vasculaire selon l'une quelconque des revendications 1 à 7, dans lequel l'axe s'étendant perpendiculairement à l'axe longitudinal est un axe parmi un axe transversal, un axe latéral ou un axe s'étendant à un angle entre ceux-ci.

9. Système d'accès vasculaire selon l'une quelconque des revendications 1 à 8, dans lequel la goupille de verrouillage (160) s'engage dans une ouverture de verrouillage (162), disposée dans l'orifice, lorsqu'elle est dans la position verrouillée.

10. Système d'accès vasculaire selon l'une quelconque des revendications 1 à 9, dans lequel la goupille de verrouillage (160) s'engage dans un élément parmi le connecteur (150) ou l'orifice (110) selon un engagement parmi un ajustement avec serrage, un ajustement forcé ou un encliquetage.

11. Système d'accès vasculaire selon l'une quelconque des revendications 1 à 10, dans lequel la goupille de verrouillage (160) dans la position verrouillée fournit un profil extérieur lisse par rapport au connecteur (150).

12. Système d'accès vasculaire selon l'une quelconque des revendications 1 à 11, dans lequel l'orifice (110) inclut un septum pénétrable par aiguille (116) disposé sur le réservoir (114) et configuré pour fournir un accès percutané au réservoir (114).

13. Système d'accès vasculaire selon l'une quelconque des revendications 1 à 12, dans lequel une surface du connecteur (150) à partir de laquelle s'étend la tige, forme un profil extérieur continu avec une surface de l'orifice (110) lorsque le connecteur est dans la position engagée.
